# EUROPEAN PATENT APPLICATION

(11) **EP 4 635 507 A1**
(43) Date of publication of application: **22.10.2025**
(21) Application number: 24171086.2
(22) Date of filing: 18.04.2024
(51) Int. Cl.: A61K 36/185, A61K 31/593, A61K 33/24, A61K 31/56, A61P 3/10

(54) **GLYCAEMIA REGULATING COMPOSITIONS**

(71) Applicant: Carbet Medica Srl, 20122 Milano (IT)
(72) Inventor: CARRABETTA, Maria Elena, 20122 MILANO (IT)
(74) Representative: Bianchetti & Minoja with Trevisan & Cuonzo IPS SRL

(57) **Abstract**

Disclosed are oral compositions whose only ingredients comprise *Lagerstroemia speciosa* dried leaf extract titrated in corosolic acid, *Punica granatum* dried extract, chromium and vitamin D3, which are useful as support for therapeutic modifications to lifestyles in order to improve the baseline blood- glucose level and insulin resistance in individuals predisposed to developing type 2 diabetes.

## Description

The object of the present invention is oral compositions comprising *Lagerstroemia speciosa* dried leaf extract titrated in corosolic acid and ellagitannins, *Punica granatum* dried extract, chromium and vitamin D3, and their use as glycaemia regulators. The compositions according to the invention are useful in particular as support for therapeutic modifications to lifestyles in order to improve the baseline blood-glucose level and insulin resistance in individuals predisposed to developing type 2 diabetes.

### Prior art

Prediabetes is an extremely frequent condition among the general population (glucose intolerance alone is estimated to affect 10% of the European population), especially in overweight adults, as it is an important component of metabolic syndrome and a risk factor for the development of type 2 diabetes and cardiovascular disease. The European guidelines suggest considering the use of prepharmacological means to optimise fasting glucose and impaired glucose tolerance (Rydén L et al., Eur Heart J. 2013 Oct; 34(39):3035-87). This type of approach is highly effective, especially in the short term (Kerrison G, et al., J Diabetes Res. 2017;2017:8493145).

However, compliance with the modified lifestyle is not usually high, except in research projects or organised protocols, and numerous patients are unable to comply with the increased physical activity requirement due to physical impairments (especially rehabilitation and/or orthopaedic problems, but also cardiovascular comorbidities, and the use of medicaments that impair the sense of balance). In this context, some substances of natural origin have proved to contribute to optimising carbohydrate metabolism when combined with suitable correction of dietary habits (Cicero AF, et al., Curr Vasc Pharmacol. 2014; 12(4):565-71).

Numerous substances of natural origin, such as cinnamon, curcumin and berberine, can contribute to improving insulin resistance. Their main limitations are usually correlated with the need for high doses repeated several times a day, with a consequent increase in costs and risk of poor compliance with the treatment.

The leaf extract of *Lagerstroemia speciosa L.* (banaba), a semideciduous tree widespread in South-East Asia, seems to perform a specific antidiabetic action in humans (Judy WV et al., J Ethnopharmacol. 2003; 87:115-117; Ikeda Y et al., Jpn Pharmacol Ther. 1999; 27:67-73). Said action has been extensively documented in preclinical studies in favour of both corosolic acid and ellagitannins (ellagic acid derivatives), water-soluble compounds which, in particular, have proved to activate glucose transport through the cell membranes in rat adipocytes, with an insulin-like effect (Hayashi T et al (2002), Planta Med 68(2) 173-175). However, unlike insulin, banaba extracts do not induce adipocyte differentiation, but inhibit it to a dose-dependent extent (Liu F et al., J Nutr. 2001; 131:2242-2247).

Said inhibition results from a reduction of PPARgamma2 (peroxisome proliferator-activated receptor gamma 2) and transport protein 4 (GLUT4) in the cells (Park M et al., (2005) Life Sci 77(26) 3344- 3354). The hypoglycaemic property and the absence of adipogenic effects have been confirmed experimentally in mice (Kakuda T et al., Biosci. Biotechnol. Biochem. 1996;60:204-204; Suzuki Y et al. JNutr Sci Vitaminol (Tokyo) 1999; 45:791-795), and appear to make banaba extracts particularly useful in moderate type 2 diabetes, even at moderate doses. Further studies have also demonstrated the effects of ellagitannins on inhibition of alpha amylase and alpha glucosidase *in vitro,* said enzymes being involved in assimilation of sugars, and therefore indirectly in increasing blood glucose.

A review of the pharmacological properties of banaba extract is reported in Miura T et al., Evidence-Based Complementary and Alternative Medicine Volume 2012, Article ID 871495.

Doses ranging from 16 to 48 mg of dried extract, titrated to 1% in corosolic acid, have been used in clinical practice (September 2000, LE Magazine). A blood glucose reduction of 4.9% was observed at the dose of 16 mg, 10.7% at 32 mg and 31.9% at 48 mg.

A second group of subjects consisting of 5 volunteers received hard gelatin capsules based on dried extract titrated to 1% in corosolic acid at the same doses as used in the preceding study. Once again, the results demonstrated a reduction in the blood glucose level, albeit less significant than the reduction obtained in the preceding study: 3.18%, 6.5% and 20.2% for the doses of 16, 32 and 48 mg respectively. The results of said study seem to demonstrate that corosolic acid is more active if administered in the form of an oily preparation.

The clinical activity of a composition of banaba aqueous extract, green tea, green coffee and garcinia was studied by Ikeda et al. in Japanese Pharmacology and Therapeutics, vol. 27, no. 5, pp. 72-73, 1999. The reduction in the blood glucose levels of 24 type II diabetics amounted to 13.5% after administration of three tablets three times a day. However, the study did not enable a distinction to be made between the contributions made by the various ingredients of the combination to the effect observed.

Lieberman et al. (Alternative and Complementary Therapies, vol. 11, no. 6, pp. 307-313, 2005) studied the effect of a diet supplement comprising 16 mg of banaba extract, 100 mg of bitter melon extract, 133 mg of Gymnema extract, 1500 mg of *Garcinia cambodia* extract, 2.6 mg of bioperine, 10 mg of an amylase inhibitor, 167 mcg of chromium, 50 mcg of vanadium and 50 mg of magnesium. After 12 weeks' treatment a significant reduction in average body weight was observed, together with a reduction in the fatty mass of the treated subjects. Once again, the contributions made by the individual ingredients of the combination studied cannot be evaluated, in particular as regards the corosolic acid and ellagitannins present in the banaba extract.

Capsule formulations of a *Lagerstroemia speciosa L.* extract containing 1% corosolic acid are known from US 7713546.

Moreover, a study published in Pharmanutrition in 2017 shows the hypoglycaemic activity of a combination of corosolic acid, ellagitannins and chromium in a defined ratio on 40 type 2 diabetics.

Thus although the glycaemia-regulating effect of banaba extract is generically known, no reliable information is available about the criticality of its composition for the purpose of hypoglycaemic activity, or the corresponding effective doses. On the basis of the available evidence, it also appears that banaba extract alone is ineffective unless associated with other extracts (such as garcinia, green tea, green coffee, Gymnema, etc.). Vitamin D3 or cholecalciferol regulates the calcium metabolism, in particular by stimulating gastrointestinal absorption of calcium. A calcium deficiency can give rise to pathological conditions such as rickets, osteomalachia, immune system disorders and neuropsychiatric disorders such as dementia, multiple sclerosis, Parkinson's disease, epilepsy, schizophrenia and depression. No study has ever been conducted to investigate the prediabetic or hypoglycaemic properties of cholecalciferol and other forms of vitamin D.

The need is particularly felt for compositions which improve the baseline blood glucose level, in particular in overweight individuals predisposed to develop type 2 diabetes, have a limited number of active ingredients with a defined, reproducible composition, and meet the regulatory requirements governing nutraceutical and diet supplements.

These and other aims are achieved by the compositions according to the invention.

### Description of the invention

It has now been discovered that improved blood-glucose control can be obtained by combining dried extract of *Lagerstroemia speciosa* leaves titrated in corosolic acid and ellagitannins, dried extract of *Punica granatum* fruit, chromium and vitamin D3. It has been found in particular that adding dried extract of *Punica granatum* fruit titrated in ellagitannins (punicalagin and ellagic acid) advantageously increases the dose of ellagitannins, which can be standardised reproducibly by a selective method.

Vitamin D3, which is involved in regulating the innate immune response, has proved to act on PPAR gamma2 (peroxisome proliferator-activated receptor-gamma2), the key factor in the cellular and nuclear inflammatory state. PPAR is responsible for numerous inflammatory processes, such as insulin resistance, autoimmune disorders, irritable bowel syndrome and metabolic syndrome.

It has been noted that the combination of *Lagerstroemia speciosa* extract, chromium, vitamin D3 and *Punica granatum* extract produces an unexpected synergistic effect. The presence of *Punica granatum* extract, a source of hydrolysable tannins, confers astringent and sequestering properties able to precipitate the carbohydrate and protein substances deriving from an exogenous diet, thereby preventing their absorption, and therefore insulin incretion. The combination according to the invention, as well as stimulating the blood-insulin receptors, provides substances able to limit absorption of the nutrients responsible for the body's blood-glucose response, thus ensuring an amplified effect. Moreover, the presence of vitamin D3 (cholecalciferol) inhibits the innate immune response, and therefore the production of pro-inflammatory enzymes and cytokines which can predispose an individual to diabetes.

The object of the invention is therefore oral compositions whose only ingredients comprise *Lagerstroemia speciosa* dried leaf extract titrated in corosolic acid, *Punica granatum* dried extract titrated in ellagitannins, chromium (III) and vitamin D3.

The formulations according to the invention promote dose-dependent improved efficacy in the treatment of prediabetic syndrome, prevention of diabetes, and treatment of insulin resistance with an inflammatory etiology.

The *Lagerstroemia speciosa L.* dried leaf extract is preferably obtained by macerating the leaves in demineralised water at 25-30°C for 1 hour in several successive stages, and extracting with water at 95-98°C and subsequently with ethanol. The extract is then concentrated under vacuum at 65-70°C, dried with spray-drying technology and finally micronised. The extract is standardised for ellagitannin content to 5-7% (HPLC method), and corosolic acid >1% (HPLC method).

The *Punica granatum* extract is preferably obtained by water-alcohol extraction of the fruit, then dried by spray-drying and finally micronised.

The weight intervals of the ingredients of the formulations according to the invention are as follows:
a) *Lagerstroemia speciosa* dried extract 50 to 100 mg,
b) *Punica granatum* dried extract 100 to 1000 mg,
c) vitamin D3 5 µg to 100 µg, amounting to 2.2 mg and 44 mg of vitamin D3 100,000 IU/g respectively,
d) chromium picolinate 0.33 mg to 2.083 mg.

The active ingredient content of a preferred composition is typically as follows: corosolic acid 0.5 mg, ellagitannins 40 mg, chromium 0.04 mg and vitamin D3 0.05 mg, in the weight ratio of 1:80:0.08:0.1 respectively

The compositions according to the invention can be in the form of tablets, hard or softgel capsules, granules or powders.

The tablets can be simple, coated, film-coated, layered, gastroresistant, slow-release (retard), orobuccal or sublingual-release tablets.

A particularly preferred form consists of tablets containing 78 mg of *Lagerstroemia speciosa L.* leaf extract with an ellagitannin content of 5-7% and a corosolic acid content of 1%, 0.33 mg of chromium picolinate, 100 mg of *Punica granatum* dried extract, and 50 µg of vitamin D3.

The compositions according to the invention, administered one to three times a day, reduce the baseline blood-glucose level and improve insulin resistance in individuals predisposed to developing type 2 diabetes.

### Example 1 - Qualitative and quantitative composition of 250 mg tablets.

| | |
|---|---|
| Microcrystalline cellulose | 54.65 mg |
| Banaba (*Lagerstroemia speciosa*) leaves, 1% corosolic acid and 5-7% ellagitannins | 50.00 mg |
| Pomegranate (*Punica granatum*) fruit extract titrated to 40% in total ellagitannins | 100 mg |
| Crospovidone | 10.00 mg |
| Sodium croscarmellose | 7.50 mg |
| Coating agents | 2.50 mg |
| Silicon dioxide | 2.00 mg |
| Magnesium stearate | 1.00 mg |
| Chromium picolinate | 0.35 mg |
| Vitamin D3 | 50 µg |

The tablets are characterised by release into the gastric environment within less than 90 minutes, according to the disintegration method described in Ph. Eur., current ed..

## Claims

1. Oral compositions whose only ingredients comprise *Lagerstroemia speciosa* dried leaf extract titrated in corosolic acid, *Punica granatum* dried extract titrated in ellagitannins, chromium (III) and vitamin D3.

2. Compositions according to claim 1 wherein the *Lagerstroemia speciosa L.* extract has a percentage content by weight of at least 1% corosolic acid and 5-7% (HPLC method) ellagitannins.

3. Compositions according to claim 1 or 2 wherein the chromium (III) is in the form of picolinate.

4. Compositions according to claim 3 wherein the *Punica granatum* extract has an ellagitannin content of 40% of the weight of the extract.

5. Compositions according to any one of claims 1 to 4 in the form of tablets comprising 50 mg of *Lagerstroemia speciosa L.* extract, 0.35 mg of chromium picolinate, 100 mg of *Punica granatum* dried extract titrated in ellagitannins, and 50 µg of vitamin D3.

6. Compositions according to the preceding claims, in the form of tablets, hard or softgel capsules, granules or powders.

7. Compositions according to claim 6 in the form of simple, coated, film-coated, layered, gastroresistant, slow-release, orobuccal or sublingual release tablets.

8. Composition according to claim 7 in the form of a gastroresistant tablet.

9. Compositions according to claim 1 for use as glycaemia-reducing or glycaemia-regulating compositions or for the treatment of prediabetes or diabetes.
